# EUROPEAN PATENT APPLICATION

(11) **EP 4 748 373 A1**
(43) Date of publication of application: **27.05.2026**
(21) Application number: 24844647.8
(22) Date of filing: 13.07.2024
(51) Int. Cl.: A61K 9/14, A61K 9/20, A61K 9/48, A61K 31/454, A61K 31/4545, A61K 31/4155, A61K 47/38, A61K 47/32, A61J 3/02, A61P 35/00, A61P 1/16, A61P 17/10

(54) **PHARMACEUTICAL COMPOSITION CONTAINING HETEROCYCLIC COMPOUND**

(30) Priority: 21.07.2023 CN 202310906213; 10.05.2024 CN 202410581506
(71) Applicant: Sagimet Biosciences Inc., San Mateo, California 94402 (US)
(72) Inventor: WU, Jinzi Jason, Hangzhou, Zhejiang 311202 (CN); DONG, Kunhua, Hangzhou, Zhejiang 311202 (CN); KEMBLE, George, San Mateo, California 94402 (US); O'FARRELL, Marie, San Mateo, California 94402 (US); WAGMAN, Allan, San Mateo, California 94402 (US)
(74) Representative: Bassil, Nicholas Charles
(86) International application number: PCT/CN2024/105370
(87) International publication number: WO 2025/020955

(57) **Abstract**

Disclosed in the present invention is a pharmaceutical composition containing a heterocyclic compound. The pharmaceutical composition comprises the following components: (a) a compound shown as formula (I); and (b) hypromellose acetate succinate or copovidone, wherein the component (a) and component (b) are mixed and subjected to hot melt extrusion, and the weight ratio of the component (b) to the component (a) is 0.4-0.8. The pharmaceutical composition disclosed by the present invention can be stably stored for long time under the condition of room temperature and relatively high humidity, and has a remarkably better dissolution effect.

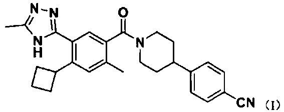

## Description

### Technical Field

The present invention relates to the technical field of pharmaceutical formulations, and in particular, to a pharmaceutical composition containing a heterocyclic compound.

### Background

The storage conditions of a drug product reflect the stability of the drug substance therein. Drugs or crystalline forms with lower melting points generally have relatively poor stability and require storage at lower temperatures, while drugs or crystalline forms with higher melting points have better stability and can generally be stored at room temperature. The appropriate processing temperature for a formulation should also be reasonably determined based on the thermal stability of the drug substance. The thermal degradation of drugs is usually closely related to their melting point, and degradation reactions occur rapidly at temperatures exceeding this point by 20°C. Both fatty liver and cancer require long-term medication. The requirement for low-temperature storage will add a lot of inconvenience to patients taking medication for a long time, and may lead to missed doses or improper storage, which could affect therapeutic efficacy and potentially cause delays or recurrence of the condition. In addition, low-temperature refrigeration conditions also necessitate dedicated cold-chain transportation equipment and long-term use of refrigerators, which brings additional costs to the commercial development of the product. Therefore, in order to better meet clinical and commercial needs, it is necessary to find a stable drug formulation capable of storage at room temperature and a preparation method therefor. Such a formulation will not only greatly increase the in vivo and in vitro dissolution of the active ingredients, but more importantly, enable its storage at room temperature. The compound of formula (I), having the generic name Denifanstat, is a potent, safe, selective oral small-molecule inhibitor of fatty acid synthase with potential for use in fatty liver and cancer research. Its chemical name is 4-[1-[4-cyclobutyl-2-methyl-5-(5-methyl-1H-1,2,4-triazol-3-yl)benzoyl]piperidin-4-yl]benzonitrile, with the molecular formula C₂₇H₂₉N₅O, the structural formula of which is as follows:

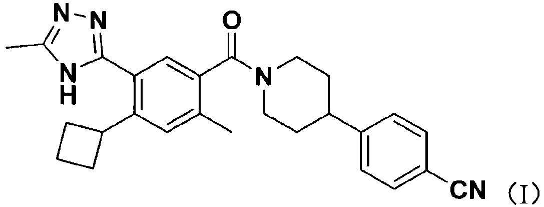

The compound shown in formula (I) exhibits poor solubility in different media (pH=1 to 7) and undergoes phase transition under conditions of high relative humidity.

Therefore, there remains a need to develop a formulation that will allow a pharmaceutical composition comprising the compound of formula (I) to be stably stored for long time under the condition of room temperature and relatively high humidity, and to have a remarkably better dissolution effect.

### Summary of the Invention

Based on this, the present invention provides a pharmaceutical composition containing a heterocyclic compound, the pharmaceutical composition comprising the following components:
(a) a compound of formula (I) 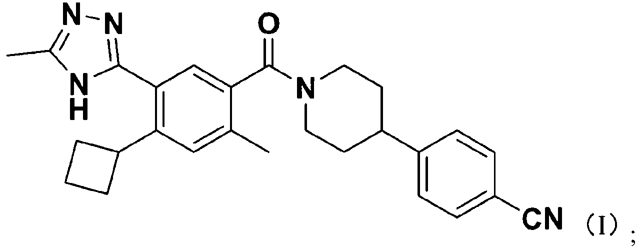 and
(b) hypromellose acetate succinate or copovidone;

wherein component (a) and component (b) are mixed and subjected to a hot-melt extrusion or spray drying process;
wherein the weight ratio of component (b) to component (a) is from 0.4 to 0.8.

Further, components (a) and (b) are mixed and subjected to hot-melt extrusion or spray drying at a temperature ranging from 145°C to 200°C. Further, components (a) and (b) are mixed and subjected to hot-melt extrusion or spray drying at a temperature ranging from 155°C to 180°C. Further, components (a) and (b) are mixed and subjected to hot-melt extrusion or spray drying at a temperature ranging from 155°C to 165°C. Further, the hypromellose acetate succinate is hypromellose acetate succinate of HG grade. Further, the weight ratio of component (b) to component (a) is from 0.5 to 0.7. Further, the weight ratio of component (b) to component (a) is about 0.66.

Further, based on the total weight of the pharmaceutical composition, the pharmaceutical composition further comprises from 50% to 70% by weight, for example from 55% to 65% by weight, of one or more pharmaceutically acceptable adjuvants, wherein the pharmaceutically acceptable adjuvants are selected from one or more of the following: water-soluble filler, water-insoluble filler, disintegrant, glidant, and lubricant. Further, the water-soluble filler is mannitol, for example mannitol M100. Further, the water-insoluble filler is microcrystalline cellulose, for example microcrystalline cellulose PH102. Further, the disintegrant is croscarmellose sodium. Further, the glidant is colloidal silicon dioxide, for example colloidal silicon dioxide M5P. Further, the lubricant is magnesium stearate, for example magnesium stearate 2257. Further, based on the total weight of the pharmaceutical composition, the pharmaceutical composition further comprises from 30% to 35% by weight of a water-soluble filler, for example about 33% by weight. Further, based on the total weight of the pharmaceutical composition, the pharmaceutical composition further comprises from 20% to 25% by weight of a water-insoluble filler, for example about 22% by weight. Further, based on the total weight of the pharmaceutical composition, the pharmaceutical composition further comprises from 0.2% to 1% by weight of a disintegrant, for example about 0.5% by weight. Further, based on the total weight of the pharmaceutical composition, the pharmaceutical composition further comprises from 1% to 3% by weight of a glidant, for example about 1.5% by weight. Further, based on the total weight of the pharmaceutical composition, the pharmaceutical composition further comprises from 1% to 3% by weight of a lubricant, for example about 1.5% by weight.

Further, the dosage form of the pharmaceutical composition is a tablet, granule, or capsule. Further, the pharmaceutical composition further comprises one or more drugs and/or extracts. Further, the drug is an anti-fatty liver drug, an anticancer drug, and/or an anti-acne drug.

According to another aspect of the present invention, a method for preparing the aforementioned pharmaceutical composition is provided, the method comprising the following steps: (1) mixing sieved component (a) with dried, milled, and sieved component (b) to obtain a mixture; (2) extruding the mixture via hot-melt extrusion at a hot-melt extrusion temperature of 145°C to 200°C to form an extrudate, or dissolving the mixture in an organic solvent, such as ethanol, dichloromethane, etc., for spray drying; (3) cooling the extrudate or spray-dried material; and (4) subjecting the cooled extrudate or spray-dried material to milling, sizing, and sieving to obtain granules of the pharmaceutical composition.

Further, the method further comprises the step of: (5) mixing and drying the granules with one or more sieved pharmaceutically acceptable adjuvants, and processing into a tablet or capsule.

Further, the mixing comprises first performing a first mix of the glidant and a portion of the water-insoluble filler; then adding the granules, the water-soluble filler, the disintegrant, and the remaining water-insoluble filler for a second mix; and finally adding the lubricant for mixing.

Further, the component (b) is hypromellose acetate succinate of HG grade. Further, the water-soluble filler is mannitol, for example mannitol M100. Further, the water-insoluble filler is microcrystalline cellulose, for example microcrystalline cellulose PH102. Further, the disintegrant is croscarmellose sodium. Further, the glidant is colloidal silicon dioxide, for example colloidal silicon dioxide M5P. Further, the lubricant is magnesium stearate, for example magnesium stearate 2257.

Further, a twin-screw hot-melt extrusion apparatus is used to extrude the mixture of components (a) and (b), wherein the screw diameter of the twin-screw hot-melt extrusion is between 8 mm and 50 mm, and the extrusion speed is between 10 rpm and 300 rpm. Further, the screw diameter of the twin-screw hot-melt extrusion is 18 mm. Further, the extrusion speed is from 75 rpm to 125 rpm, for example about 100 rpm.

Further, steps (1) to (5) of the method comprise any one or more of the following items [1] to [28]: [1] the loss on drying determination in step (1) shows a result of not more than 2%; [2] the drying temperature in step (1) is 55°C to 65°C, for example about 60°C; [3] the milling speed in step (1) is 5000 rpm to 6000 rpm, for example about 5500 rpm; [4] the sieve in step (1) is a 30-mesh sieve or a 40-mesh sieve; [5] the mixing time in step (1) is 10 min to 20 min, for example about 15 min; [6] the mixing speed in step (1) is 15 rpm to 25 rpm, for example about 20 rpm; [7] the temperature of zone 1 of the extruder in step (2) is 70°C to 110°C, for example about 90°C; [8] the temperature of zone 2 of the extruder in step (2) is 110°C to 150°C, for example about 130°C; [9] the temperature of zone 3 of the extruder in step (2) is 155°C to 165°C, for example about 160°C; [10] the temperatures of zones 4-10 of the extruder in step (2) are 155°C to 165°C, for example about 160°C; [11] the temperature of zone 11 of the extruder in step (2) is 140°C to 180°C, for example about 160°C; [12] the die temperature of the extruder in step (2) is 150°C to 170°C, for example about 160°C; [13] the melt pressure of the extruder in step (2) is not more than 150 bar; [14] the load pressure of the extruder in step (2) is not more than 80%, for example 25% to 65%; [15] the cooling belt speed of the extruder in step (2) is 1 m/min to 10 m/min; [16] the processing rate of the extruder in step (2) is 0.1 kg/h to 10 kg/h; [17] the sieve aperture for milling in step (4) is about 2 mm; [18] the milling frequency in step (4) is 35 Hz to 45 Hz, for example about 40 Hz; [19] the milling speed in step (4) is 5000 rpm to 6000 rpm, for example about 5500 rpm; [20] the rotation speed for sizing in step (4) is 5000 rpm to 6000 rpm, for example about 5500 rpm; [21] the sieve aperture for sizing in step (4) is about 0.8 mm; [22] the sieve in step (4) is a 40-mesh sieve; [23] the particle size of the granules in step (4) is not more than 250 µm, for example 100 µm to 250 µm; [24] the time for the first mix in step (5) is 10 min to 20 min, for example about 15 min; [25] the speed for the first mix in step (5) is 15 rpm to 25 rpm, for example about 20 rpm; [26] the time for the second mix in step (5) is 2 min to 6 min, for example about 4 min; [27] the speed for the second mix in step (5) is 15 rpm to 25 rpm, for example about 20 rpm; [28] the loss on drying determination in step (5) shows a result of not more than 5%.

The present invention provides a pharmaceutical composition containing a heterocyclic compound, the pharmaceutical composition comprising the following components:
(a) a compound of formula (I) 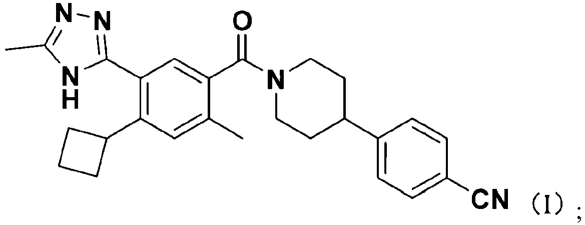 and
(b) a dispersion polymer;

wherein component (a) and component (b) are mixed at a temperature ranging from 140°C to 250°C;
wherein the weight ratio of component (b) to component (a) is from 0.4 to 0.8.

Further, the dispersion polymer is hypromellose acetate succinate or copovidone.

Further, component (a) and component (b) are mixed via hot-melt extrusion.

Further, components (a) and (b) are mixed and subjected to hot-melt extrusion or spray drying at a temperature ranging from 145°C to 200°C. Further, components (a) and (b) are mixed and subjected to hot-melt extrusion or spray drying at a temperature ranging from 155°C to 180°C. Further, components (a) and (b) are mixed and subjected to hot-melt extrusion or spray drying at a temperature ranging from 155°C to 165°C.

Further, the hypromellose acetate succinate is hypromellose acetate succinate of HG grade.

Further, the weight ratio of component (b) to component (a) is from 0.5 to 0.7.

Further, the weight ratio of component (b) to component (a) is about 0.66.

Further, based on the total weight of the pharmaceutical composition, the pharmaceutical composition further comprises from 50% to 70% by weight, for example from 55% to 65% by weight, of one or more pharmaceutically acceptable adjuvants, wherein the pharmaceutically acceptable adjuvants are selected from one or more of the following: water-soluble filler, water-insoluble filler, disintegrant, glidant, and lubricant. Further, the water-soluble filler is mannitol, for example mannitol M100. Further, the water-insoluble filler is microcrystalline cellulose, for example microcrystalline cellulose PH102. Further, the disintegrant is croscarmellose sodium. Further, the glidant is colloidal silicon dioxide, for example colloidal silicon dioxide M5P. Further, the lubricant is magnesium stearate, for example magnesium stearate 2257. Further, based on the total weight of the pharmaceutical composition, the pharmaceutical composition further comprises from 30% to 35% by weight of a water-soluble filler, for example about 33% by weight. Further, based on the total weight of the pharmaceutical composition, the pharmaceutical composition further comprises from 20% to 25% by weight of a water-insoluble filler, for example about 22% by weight. Further, based on the total weight of the pharmaceutical composition, the pharmaceutical composition further comprises from 0.2% to 1% by weight of a disintegrant, for example about 0.5% by weight. Further, based on the total weight of the pharmaceutical composition, the pharmaceutical composition further comprises from 1% to 3% by weight of a glidant, for example about 1.5% by weight. Further, based on the total weight of the pharmaceutical composition, the pharmaceutical composition further comprises from 1% to 3% by weight of a lubricant, for example about 1.5% by weight.

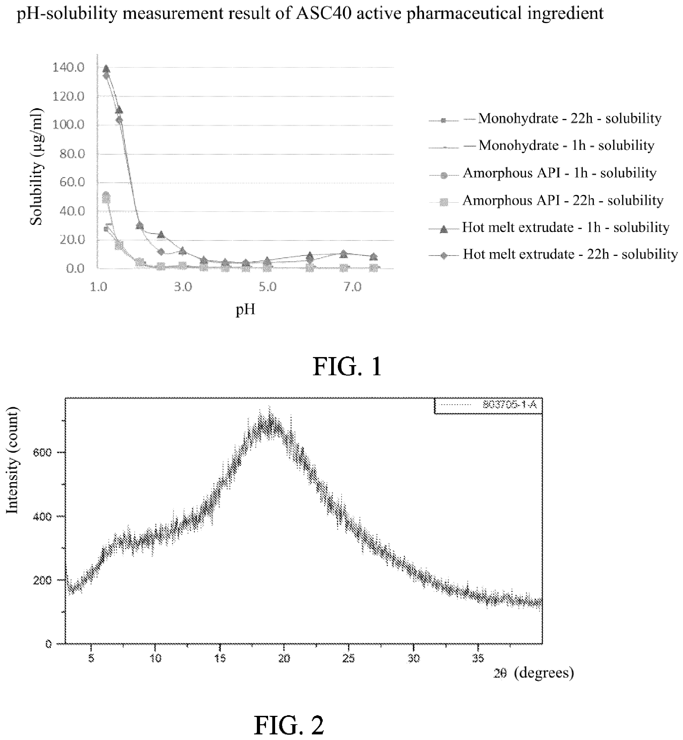

Further, the dosage form of the pharmaceutical composition is a tablet, granule, or capsule. Further, the pharmaceutical composition further comprises one or more drugs and/or extracts. Further, the compound of formula (I) is in an amorphous solid form. Further, the amorphous solid form, when analyzed thermally using differential scanning calorimetry (DSC), exhibits an endothermic peak at approximately 98°C. Further, the amorphous solid form, when analyzed thermally using modulated differential scanning calorimetry (MDSC), has a glass transition temperature of about 113°C. Further, the amorphous solid form has an x-ray powder diffraction (XRPD) pattern substantially as shown in FIG. 2. Further, the amorphous solid form is prepared as follows: a hot-melt extrudate dispersion intermediate is prepared by using specific adjuvants through a hot-melt extrusion process or a spray drying process, which process transforms the compound of formula (I) from a crystalline form into a stable, non-crystalline amorphous form.

According to another aspect of the present invention, a use of the aforementioned pharmaceutical composition in the preparation of a drug for preventing or treating fatty liver is provided. According to another aspect of the present invention, a use of the aforementioned pharmaceutical composition in the preparation of a drug for preventing or treating cancer is provided. According to another aspect of the present invention, a use of the aforementioned pharmaceutical composition in the preparation of a drug for preventing or treating acne is provided.

The beneficial effects of the present invention are:
The present invention uses the free base monohydrate crystalline form of the compound of formula (I) to produce the pharmaceutical composition of the present invention. To improve the dissolution rate of the compound of formula (I) and promote consistency of oral absorption, the adjuvant hypromellose acetate succinate-HG (HPMCAS-HG, a dispersion polymer) is used to prepare a hot-melt extrudate dispersion intermediate (HMEDI) via a hot-melt extrusion process. This process transforms the compound of formula (I) from the free base monohydrate crystalline form to a stable, non-crystalline amorphous form. The pharmaceutical composition disclosed by the present invention can be stably stored for long time under the condition of room temperature and relatively high humidity, and has a remarkably better dissolution effect.

The pharmaceutical composition disclosed by the present invention can be stably stored for long time under the condition of room temperature and relatively high humidity, and has a remarkably better dissolution effect.

### Description of the Drawings

To illustrate the technical solutions in the embodiments of the present invention more clearly, the drawings used in the description of the embodiments will be briefly introduced below. Obviously, the drawings described below are only some embodiments of the present invention. For those skilled in the art, other drawings may be derived from these drawings, without departing from the scope of protection sought by the present invention.
FIG. 1 is a schematic diagram showing the results of the equilibrium solubility experiments in buffer media at different pH conditions for the monohydrate crystalline form of the compound of formula (I), the amorphous form of the compound of formula (I), and the hot-melt extrudate dispersion intermediate of the present invention.
FIG. 2 is the X-ray powder diffraction (XRPD) pattern of the amorphous form of the compound of formula (I).

### Detailed Description

The technical solutions in the embodiments of the present invention will be described clearly and completely below in conjunction with the drawings in the embodiments of the present invention. Obviously, the described embodiments are some, but not all, of the embodiments of the present invention. Based on the embodiments of the present invention, all other embodiments obtained by those skilled in the art without creative efforts shall fall within the scope of protection of the present invention.

Unless otherwise stated, all technical and scientific terms and abbreviations used herein have the meanings commonly understood by one of ordinary skill in the field of the present invention or in the field where the term is applied. While any methods, conditions, substances, or materials similar to or equivalent to those disclosed herein may be used in the practice of the present invention, preferred methods, conditions, substances, or materials are described herein.

The present invention is intended to encompass all alternatives, variations and equivalents that may be included within the scope of the invention as defined by the claims. Those skilled in the art will recognize many methods and substances similar or equivalent to those described herein, which can be applied in the practice of the present invention. The present invention is in no way limited to the description of the methods and substances.

The singular forms "a," "an," and "the" used in the specification and the appended claims include plural referents unless the context clearly indicates otherwise.

In the present invention, the term "comprises" is synonymous with "includes." The terms "comprising," "including," "having," "containing," or any other variations thereof, as used herein, are intended to cover non-exclusive inclusion. For example, a composition, step, method, article, or device comprising listed elements is not necessarily limited to those elements, but may include other elements not explicitly listed or those inherent to this composition, step, method, article, or device.

As described in the Background section, the compound of formula (I) suffers from poor solubility in different media (pH=1 to 7) and undergoes phase transition under conditions of high relative humidity. To address the aforementioned problems, the present invention provides a pharmaceutical composition containing a heterocyclic compound, the pharmaceutical composition comprising the following components:
(a) a compound of formula (I) 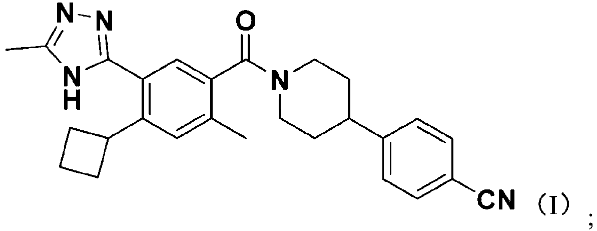 and
(b) hypromellose acetate succinate or copovidone;

wherein component (a) and component (b) are mixed and subjected to hot-melt extrusion or spray drying;
wherein the weight ratio of component (b) to component (a) is from 0.4 to 0.8.

In the tablet development of the present invention, three batches of the active pharmaceutical ingredient of the compound of formula (I) were studied, all in the form of a monohydrate crystalline form (for the synthesis method, refer to the synthesis of ASC40 in WO2012122391A1). After hot-melt extrusion, in the solid dispersion (HMEDI) prepared from a physical mixture containing the compound of formula (I) in the monohydrate crystalline form (60% by mass) and HPMCAS-HG (40% by mass), all characteristic crystalline form diffraction peaks disappeared, and it was in an amorphous state.

Since the performance of the formulation depends on maintaining the active pharmaceutical ingredient in the non-crystalline amorphous form, in the stability study, the present invention monitored the phase transition of HMEDI from a non-crystalline amorphous form to a crystalline form by XRPD and by measuring the glass transition temperature via DSC. During the stability study, the present invention further monitored changes in the dissolution of the tablets of the present invention. Changes in the dissolution may indicate reduced formulation performance due to transformation of the amorphous form into the crystalline state. The present invention found that since the compound of formula (I) undergoes phase transition under conditions of high relative humidity, i.e., it can transform from an amorphous, non-crystalline state to a poorly soluble crystalline form, a study was conducted to investigate HMEDI under accelerated temperature and humidity conditions, in order for the compound of formula (I) to maintain in an amorphous, non-crystalline form when exposed to moist or aqueous environments (such as the gastrointestinal tract) and in order to better prevent water from inducing transformation of the compound of formula (I). XRPD analysis results showed that, under conditions of 25°C/95%RH, the milled powder of HMEDI in the open container did not undergo crystalline transformation during the 30-day study period. Under conditions of 25±2°C/60±5% RH and 40±2°C/75±5% RH, no signs of crystal formation affecting stability were observed in HMEDI packaged to prevent moisture absorption. This indicates that formulations made from HMEDI can resist phase transition in the presence of exogenous water and show potential for good stability over a longer shelf life.

In the present invention, when parts by weight, temperature, speed, diameter, time, pressure, frequency, proportion, equivalent, concentration, or other values or parameters are expressed as a range, a preferred range, or a range defined by a series of upper limit preferred values and lower limit preferred values, it should be understood that all ranges formed by any pairing of any upper range limit or preferred value with any lower range limit or preferred value are specifically disclosed, regardless of whether such ranges are separately disclosed. For example, when the range "0.4 to 0.8" is disclosed, the described range should be interpreted as including the ranges "0.4 to 0.8", "0.4 to 0.7", "0.4 to 0.6", "0.4 to 0.5", "0.5 to 0.8", "0.5 to 0.7", "0.5 to 0.6", "0.6 to 0.8", "0.6 to 0.7", "0.7 to 0.8", "0.4 to 0.75", "0.4 to 0.65", "0.4 to 0.55", "0.4 to 0.45", "0.5 to 0.75", "0.5 to 0.65", "0.5 to 0.55," etc. When a numerical range is described herein, unless otherwise stated, the range is intended to include its endpoints and all values to two decimal places within the range, and the effects of the present invention can be achieved within the aforementioned ranges.

In a preferred embodiment, based on the total weight of the pharmaceutical composition, the pharmaceutical composition comprises from 20% to 30% by weight of component (a), for example about 24% by weight. In a preferred embodiment, based on the total weight of the pharmaceutical composition, the pharmaceutical composition comprises from 10% to 20% by weight of component (a), for example about 16% by weight.

In a preferred embodiment, components (a) and (b) are mixed and subjected to hot-melt extrusion or spray drying at a temperature ranging from 145°C to 200°C. In a preferred embodiment, components (a) and (b) are mixed and subjected to hot-melt extrusion or spray drying at a temperature ranging from 155°C to 180°C. In a preferred embodiment, components (a) and (b) are mixed and subjected to hot-melt extrusion or spray drying at a temperature ranging from 155°C to 165°C.

To better enable the compound of formula (I) of the present invention to maintain in the amorphous form, in a preferred embodiment, the hypromellose acetate succinate is hypromellose acetate succinate of HG grade.

In a preferred embodiment, the weight ratio of component (b) to component (a) is 0.5 to 0.7. In a preferred embodiment, the weight ratio of component (b) to component (a) is about 0.66.

Ranges herein may be expressed as from "about" one particular value, and/or to "about" another particular value. When expressing such a range, another embodiment includes from the one particular value and/or to the other particular value. Similarly, when a value is expressed as an approximation by use of the antecedent "about," it will be understood that the particular value forms another embodiment. It will be further understood that the endpoints of each range are significant both in relation to the other endpoint and independently of the other endpoint. It should also be understood that many values are disclosed herein, and each value is also disclosed herein as "about" that particular value in addition to the value itself. For example, if the value "10" is disclosed, then "about 10" is also disclosed. It should also be understood that when "less than or equal to" the value, "greater than or equal to the value" is disclosed, then the possible range between these values is also disclosed, as appropriately understood by those skilled in the art. For example, if the value "10" is disclosed, then "less than or equal to 10" and "greater than or equal to 10" are also disclosed.

In the present invention, "about" means a value within ±5% of a particular value. For example, "about 24" includes 24 ± 5%, or from 22.8 to 25.2; "about 16" includes 16 ± 5%, or from 15.2 to 16.8; "about 0.66" includes 0.66 ± 5%, or from 0.627 to 0.693.

In a preferred embodiment, based on the total weight of the pharmaceutical composition, the pharmaceutical composition further comprises 50% to 70% by weight, for example 55% to 65% by weight, one or more pharmaceutically acceptable adjuvants, wherein the pharmaceutically acceptable adjuvants are selected from one or more of the following: water-soluble filler, water-insoluble filler, disintegrant, glidant, and lubricant.

In the present invention, the term "pharmaceutically acceptable" refers to a substance, such as a carrier or diluent, that does not abolish the biological activity or properties of the compound, and is relatively non-toxic, i.e., administration of the substance to an individual does not cause undesirable biological effects or interact in a harmful manner with any components contained therein.

In the present invention, the term "pharmaceutically acceptable adjuvants" refers to carriers and/or excipients that are pharmacologically and/or physiologically compatible with the subject and the active ingredient (i.e., capable of eliciting the desired therapeutic effect without causing any undesirable local or systemic effects), which are well known in the art (see, for example, Remington's Pharmaceutical Sciences. Edited by Gennaro AR, 19th ed. Pennsylvania: Mack Publishing Company, 1995).

To better function as a water-soluble filler to aid wetting and capillary action and to better improve the tableting properties of the final mixture of the present invention, compared to other water-soluble fillers, in a preferred embodiment, the water-soluble filler is mannitol, for example mannitol M100.

To better function as a water-insoluble filler to aid fragmentation of the solid and to better adjust the granulation density of the present invention, compared to other water-insoluble fillers, in a preferred embodiment, the water-insoluble filler is microcrystalline cellulose, for example microcrystalline cellulose PH102.

To better aid disintegration and fragmentation of the tablet solid of the present invention, compared to other disintegrants, in a preferred embodiment, the disintegrant is croscarmellose sodium.

To better promote powder flow of the present invention, compared to other glidants, in a preferred embodiment, the glidant is colloidal silicon dioxide, for example colloidal silicon dioxide M5P.

To better prevent caking and adhesion phenomena in the production equipment for the pharmaceutical composition of the present invention, compared to other lubricants, in a preferred embodiment, the lubricant is magnesium stearate, for example magnesium stearate 2257.

The aforementioned adjuvants are preferably pharmaceutically inert, or may have synergistic or additive effects to enhance the therapeutic activity of the pharmaceutical composition. The aforementioned adjuvants are merely exemplary, and the adjuvants actually used in the present invention are not limited to the above and may be adjusted according to actual situations, all of which can achieve the effects of the present invention.

To enhance the compressibility of the granules of the present invention, in a preferred embodiment, based on the total weight of the pharmaceutical composition, the pharmaceutical composition further comprises from 30% to 35% by weight of a water-soluble filler, for example about 33% by weight. When the weight percentage is below 30%, the disintegration time of the tablet will be significantly prolonged; when the weight percentage is above 35%, uneven mixing of granules may occur.

To enhance the compressibility of the granules of the present invention, in a preferred embodiment, based on the total weight of the pharmaceutical composition, the pharmaceutical composition further comprises from 20% to 25% by weight of a water-insoluble filler, for example about 22% by weight. When the weight percentage is below 20%, the compressibility of the granules may be poor, resulting in low hardness; when the weight percentage is above 25%, uneven mixing of granules may occur.

To cause the granules of the present invention to show a roughly linear correlation between increasing tableting pressure, increasing tablet hardness, and increasing disintegration time, in a preferred embodiment, based on the total weight of the pharmaceutical composition, the pharmaceutical composition further comprises from 0.2% to 1% by weight of a disintegrant, for example about 0.5% by weight. When the weight percentage is below 0.2%, the disintegration time of the tablet will be significantly prolonged; when the weight percentage is above 1%, the tablet may disintegrate too quickly, potentially disintegrating in the mouth during administration.

In a preferred embodiment, based on the total weight of the pharmaceutical composition, the pharmaceutical composition further comprises from 1% to 3% by weight of a glidant, for example about 1.5% by weight. When the weight percentage is below 1%, poor granule flowability may occur; when the weight percentage is above 3%, the granule density may be too low, resulting in poor compressibility.

To reduce the ejection force of the tablets of the present invention, in a preferred embodiment, based on the total weight of the pharmaceutical composition, the pharmaceutical composition further comprises from 1% to 3% by weight of a lubricant, for example about 1.5% by weight. When the weight percentage is below 1%, sticking to the punch may occur during the tableting process; when the weight percentage is above 3%, a reduction in dissolution of the tablets may occur.

In the present invention, "about" means a value within ±5% of a particular value. For example, "about 33" includes 33 ± 5%, or from 31.35 to 34.65; "about 22" includes 22 ± 5%, or from 20.9 to 23.1; "about 0.5" includes 0.5 ± 5%, or from 0.475 to 0.525; "about 1.5" includes 1.5 ± 5%, or from 1.425 to 1.575.

In a preferred embodiment, the dosage form of the pharmaceutical composition is tablets, granules, or capsules. In a preferred embodiment, the pharmaceutical composition further comprises one or more drugs and/or extracts.

In a preferred embodiment, the drug is an anti-fatty liver drug and/or an anticancer drug.

According to another aspect of the present invention, a method for preparing the aforementioned pharmaceutical composition is provided, the method comprising the following steps: (1) mixing sieved component (a) with dried, milled, and sieved component (b) to obtain a mixture; (2) extruding the mixture via hot-melt extrusion at a hot-melt extrusion temperature of 145°C to 200°C to form an extrudate, or dissolving the mixture in an organic solvent, such as ethanol, dichloromethane, acetonitrile, etc., for spray drying; (3) cooling the extrudate or spray-dried material; and (4) subjecting the cooled extrudate or spray-dried material to milling, sizing, and sieving to obtain granules of the pharmaceutical composition.

In a preferred embodiment, the method further includes the following steps:
(5) mixing and drying the granules with one or more sieved pharmaceutically acceptable adjuvants, and processing into a tablet or capsule.

In a preferred embodiment, the mixing comprises first performing a first mix of the glidant and a portion of the water-insoluble filler; then adding the granules, the water-soluble filler, the disintegrant, and the remaining water-insoluble filler for a second mix; and finally adding the lubricant for mixing.

In a preferred embodiment, the component (b) is hypromellose acetate succinate of HG grade. In a preferred embodiment, the water-soluble filler is mannitol, for example mannitol M100. In a preferred embodiment, the water-insoluble filler is microcrystalline cellulose, for example microcrystalline cellulose PH102. In a preferred embodiment, the disintegrant is croscarmellose sodium. In a preferred embodiment, the glidant is colloidal silicon dioxide, for example colloidal silicon dioxide M5P. In a preferred embodiment, the lubricant is magnesium stearate, for example magnesium stearate 2257.

In a preferred embodiment, a twin-screw hot-melt extrusion apparatus is used to extrude the mixture of components (a) and (b), wherein the screw diameter of the twin-screw hot-melt extrusion is between 8 mm and 50 mm, and the extrusion speed is between 10 rpm and 300 rpm. In a preferred embodiment, the screw diameter of the twin-screw hot-melt extrusion is 18 mm. In a preferred embodiment, the extrusion speed is from 75 rpm to 125 rpm, for example about 100 rpm.

In the present invention, "about" means a value within ±5% of a particular value. For example, "about 100" includes 100 ± 5%, or from 95 to 105.

In a preferred embodiment, steps (1) to (5) of the method comprise any one or more of the following items [1] to [28]: [1] the loss on drying determination in step (1) shows a result of not more than 2%; [2] the drying temperature in step (1) is 55°C to 65°C, for example about 60°C; [3] the milling speed in step (1) is 5000 rpm to 6000 rpm, for example about 5500 rpm; [4] the sieve in step (1) is a 30-mesh sieve or a 40-mesh sieve; [5] the mixing time in step (1) is 10 min to 20 min, for example about 15 min; [6] the mixing speed in step (1) is 15 rpm to 25 rpm, for example about 20 rpm; [7] the temperature of zone 1 of the extruder in step (2) is 70°C to 110°C, for example about 90°C; [8] the temperature of zone 2 of the extruder in step (2) is 110°C to 150°C, for example about 130°C; [9] the temperature of zone 3 of the extruder in step (2) is 155°C to 165°C, for example about 160°C; [10] the temperatures of zones 4-10 of the extruder in step (2) are 155°C to 165°C, for example about 160°C; [11] the temperature of zone 11 of the extruder in step (2) is 140°C to 180°C, for example about 160°C; [12] the die temperature of the extruder in step (2) is 150°C to 170°C, for example about 160°C; [13] the melt pressure of the extruder in step (2) is not more than 150 bar; [14] the load pressure of the extruder in step (2) is not more than 80%, for example 25% to 65%; [15] the cooling belt speed of the extruder in step (2) is 1 m/min to 10 m/min; [16] the processing rate of the extruder in step (2) is 0.1 kg/h to 10 kg/h; [17] the sieve aperture for milling in step (4) is about 2 mm; [18] the milling frequency in step (4) is 35 Hz to 45 Hz, for example about 40 Hz; [19] the milling speed in step (4) is 5000 rpm to 6000 rpm, for example about 5500 rpm; [20] the rotation speed for sizing in step (4) is 5000 rpm to 6000 rpm, for example about 5500 rpm; [21] the sieve aperture for sizing in step (4) is about 0.8 mm; [22] the sieve in step (4) is a 40-mesh sieve; [23] the particle size of the granules in step (4) is not more than 250 µm, for example 100 µm to 250 µm; [24] the time for the first mix in step (5) is 10 min to 20 min, for example about 15 min; [25] the speed for the first mix in step (5) is 15 rpm to 25 rpm, for example about 20 rpm; [26] the time for the second mix in step (5) is 2 min to 6 min, for example about 4 min; [27] the speed for the second mix in step (5) is 15 rpm to 25 rpm, for example about 20 rpm; [28] the loss on drying determination in step (5) shows a result of not more than 5%.

Regarding "[23] the particle size of the granules in step (4) is not more than 250 µm, for example 100 µm to 250 µm", the present invention investigated the effect of particle size on the dissolution curve of hot-melt extrudate dispersion intermediate (HMEDI). The hot-melt extrudate dispersion intermediate (HMEDI) was milled and sieved to separate samples with three particle size distributions (PSD): <100 µm, 100 to 250 µm and >250 µm. The dissolution curves of HMEDI granules with particle size <100 µm and particle size 100-250 µm were similar to that of SDD; larger granules with particle size >250 µm did not dissolve smoothly and tended to agglomerate, forming a gel and resulting in a lower concentration of free compound of formula (I) under SGF and SIF conditions. Based on these results, a PSD of 100 to 250 µm with suitable dissolution characteristics was selected for further development, and tablet mixture adjuvants with similar PSD were used to support suitable mixing uniformity. The hot-melt extrudate dispersion intermediate (HMEDI) of the desired PSD was produced by milling and sieving and directly using a hammer mill (with appropriate hammering speed and sieve size). Feasibility study on tableting using these batches of hot-melt extrudate dispersion intermediates (HMEDIs) was conducted.

In the present invention, the spray-dried dispersion (SDD) in amorphous solid form improved the solubility and dissolution rate of the compound of formula (I). The dissolution results for milled powder of the hot-melt extrudate dispersion intermediate (HMEDI) were comparable to those of SDD.

In the present invention, the spray-dried dispersion (SDD) powder tends to achieve a relatively high API dissolution concentration (typically >1000 µg/mL) in acidic (approx. pH 1.2) simulated gastric fluid (SGF). When the medium is switched to the more alkaline (approx. pH 6.8) fasted state simulated intestinal fluid (FaSSIF), the concentration drops by 5-6 times. HPMCAS-HG is known as an enteric coating agent; it is insoluble in gastric fluid and dissolves immediately upon transfer to the small intestine. The high dissolution concentration of the compound of formula (I) observed in the spray-dried dispersion (SDD) may be related to the proportion of the molecules of the compound of formula (I) exposed on the surface of the polymer dispersion granules. The dissolved API in the acidic gastric environment, upon transfer from the stomach to the higher pH intestine, experiences a drop in dissolution concentration, likely because the originally ionized API precipitates from solution upon pH shift, transforming into the insoluble monohydrate crystalline form. By increasing the amount of HPMCAS-HG from 25% in the spray-dried dispersion (SDD) to 40% in the hot-melt extrudate dispersion intermediate (HMEDI), the dissolution rate of the API in acidic aqueous medium is reduced, thereby preserving the amorphous form of the active pharmaceutical ingredient for release in the intestine. The study on the dissolution behavior of representative batches of the hot-melt extrudate dispersion intermediate (HMEDI) of the compound of formula (I) during the gastric-to-intestinal transition process can substantiate the above theory.

In the present invention, "about" means a value within ±5% of a particular value. For example, "about 60" includes 60 ± 5%, or from 57 to 63; "about 5500" includes 5500 ± 5%, or from 5225 to 5775; "about 15" includes 15 ± 5%, or from 14.25 to 15.75; "about 20" includes 20 ± 5%, or from 18 to 22; "about 90" includes 90 ± 5%, or from 85.5 to 94.5; "about 130" includes 130 ± 5%, or from 123.5 to 136.5; "about 160" includes 160 ± 5%, or from 152 to 168; "about 2" includes 2 ± 5%, or from 1.9 to 2.1; "about 40" includes 40 ± 5%, or from 38 to 42; "about 0.8" includes 0.8 ± 5%, or from 0.76 to 0.84; "about 4" includes 4 ± 5%, or from 3.8 to 4.2.

In a preferred embodiment, the compound of formula (I) is in an amorphous solid form. In a preferred embodiment, the amorphous solid form, when analyzed thermally using differential scanning calorimetry (DSC), exhibits an endothermic peak at approximately 98°C. In a preferred embodiment, the amorphous solid form, when analyzed thermally using modulated differential scanning calorimetry (MDSC), has a glass transition temperature of about 113°C. In a preferred embodiment, the amorphous solid form has an x-ray powder diffraction (XRPD) pattern substantially as shown in FIG. 2. In a preferred embodiment, the amorphous solid form is prepared as follows: a hot-melt extrudate dispersion intermediate is prepared by using specific adjuvants through a hot-melt extrusion process or a spray drying process, which process transforms the compound of formula (I) from a crystalline form into a stable, non-crystalline amorphous form.

According to another aspect of the present invention, a use of the aforementioned pharmaceutical composition in the preparation of a drug for preventing or treating fatty liver is provided. According to another aspect of the present invention, a use of the aforementioned pharmaceutical composition in the preparation of a drug for preventing or treating cancer is provided. According to another aspect of the present invention, a use of the aforementioned pharmaceutical composition in the preparation of a drug for preventing or treating acne is provided.

In the present invention, the term "prevention" includes "treatment" and "prophylaxis," unless specifically indicated otherwise. The terms "therapeutic" and "therapeutically" should be construed accordingly. In the present invention, the term "treatment" includes alleviating, inhibiting, or ameliorating the symptoms or condition of a disease; inhibiting the production of complications; improving or preventing underlying metabolic syndrome; inhibiting the development of a disease or symptom, such as controlling the progression of the disease or condition; alleviating the disease or symptom; causing the disease or symptom to regress; alleviating complications caused by the disease or symptom, or preventing or treating signs caused by the disease or symptom. As used herein, a pharmaceutical composition or a pharmaceutical formulation, after administration, can improve a disease, symptom, or condition, particularly by reducing its severity, delaying its onset, slowing its progression, or reducing its duration. Situations attributable to or associated with the administration, whether fixed or provisional, continuous or intermittent, are included.

According to another aspect of the present invention, the aforementioned pharmaceutical composition is provided for use in preventing and/or treating fatty liver in a subject. According to another aspect of the present invention, the aforementioned pharmaceutical composition is provided for use in preventing and/or treating cancer in a subject. According to another aspect of the present invention, a method for preventing and/or treating fatty liver in a subject is provided, comprising administering an effective amount of the aforementioned pharmaceutical composition to the subject. According to another aspect of the present invention, a method for preventing and/or treating cancer in a subject is provided, comprising administering an effective amount of the aforementioned pharmaceutical composition to the subject. According to another aspect of the present invention, a method for preventing and/or treating acne in a subject is provided, comprising administering an effective amount of the aforementioned pharmaceutical composition to the subject.

In the present invention, the term "subject" is a mammal. The mammal can be a human, a non-human primate, mouse, rat, dog, cat, horse, or cow, but is not limited to these examples. Mammals other than humans can advantageously be used as subjects representing models of fatty liver or cancer. Preferably, the subject is a human. The "effective amount" of the pharmaceutical composition or formulation used in the present invention is an amount that achieves the desired therapeutic and/or prophylactic effect. The amount effective for this purpose will depend on, for example, the pharmaceutical composition, the mode of administration, the stage and severity of the disease being treated, the individual's body weight and overall health, and the judgment of the prescribing physician. The dosage can be administered once weekly, every other day, once daily, or even several times a day. The dosage units can be administered over a short period (e.g., weeks to months) or a longer period (months to years).

The present invention is further illustrated by the following specific examples. It should be understood that these examples are only for illustrating the present invention and not for limiting the scope of the present invention. The experimental methods in the following examples for which specific conditions are not indicated are generally carried out under conventional conditions or under conditions recommended by the manufacturer.

Unless otherwise defined, all professional and scientific terms used herein have the same meanings as commonly understood by those skilled in the art. Furthermore, any methods and materials similar or equivalent to those described may be applied in the methods of the present invention. The preferred implementation methods and materials described herein are for illustrative purposes only.

The features mentioned above in the present invention, or the features mentioned in the examples, may be combined arbitrarily. All features disclosed in this patent specification may be combined in any composition form. The various features disclosed in the specification may be replaced by any alternative features that provide the same, equivalent, or similar purpose. Therefore, unless specifically stated otherwise, the disclosed features are only general examples of equivalent or similar features.

### Examples

### 1. Investigation of hot-melt extrusion solid dispersion process

The hot-melt extrusion process involves mixing and thermally melting a mixture of 60% compound of formula (I) and 40% HPMCAS-HG under controlled temperature in the barrel of a screw-driven extruder, to produce a transparent, homogeneous amorphous extrudate. DSC and TGA analysis of the compound of formula (I) show a melting point of about 145°C, with weight loss occurring above 250°C, indicating decomposition. The DSC melting transition temperature of HPMCAS-HG is about 120°C, while TGA weight loss indicates decomposition above 200°C. Based on this thermal characterization, 160°C was selected as the processing temperature for hot-melt extrusion (HME), and an upper limit temperature of 180°C was selected to avoid decomposition of the compound of formula (I).

To improve the homogeneity of the extrudate, the compound of formula (I) and HPMCAS-HG were pre-mixed before hot-melt extrusion. Since the particle size distribution D(v,0.9) of HPMCAS-HG is typically less than about 1000 µm, its particle size distribution was reduced via a milling step to D(v,0.9) less than 250-300 µm. The particle size distribution of HPMCAS-HG was controlled to be closer to that of the compound of formula (I) (D(v,0.9) less than about 100-200 µm) to support uniformity during the mixing process.

The pre-mixed material was directly fed into the extruder barrel via the main feed port of an 18 mm twin-screw extruder. The screw configuration of the extruder is designed with an intensive mixing section, maintaining moderate shear force to avoid material degradation. The extruder was driven by screw and feed rate, with a barrel temperature of 160°C and a processing rate of about 1-2 kg/h. Process response parameters such as extrudate product temperature, extruder load pressure %, and melt pressure were monitored during the process. The load pressure exceeding 80% could shut down the extruder, while high melt pressure would lead to increased product temperature and potential product degradation. The hot-melt extrusion process parameters and target ranges for the 18 mm twin-screw extruder are shown in Table 1.

**Table 1 Hot-melt extrusion process parameters and target ranges for 18 mm twin-screw extruder**

| Hot-melt extrusion parameter | Range |
|---|---|
| Screw speed | 75-125rpm |
| Barrel temperature | 155-165°C |
| Product temperature | 155-165°C |
| Extruder load pressure % | 25-65% |

Following process parameter optimization, representative samples were collected at the beginning, middle and end of the extrusion run (0, 30, 60, 180, 360 and 390 min) during the approximately 6.5-hour extrusion process to monitor product quality. No significant changes were observed in the relevant substances in the product, and all samples were determined to be in the amorphous form. In addition, the present invention involved the control and pre-treatment of HPMCAS-HG: about 10 g of HPMCAS-HG was taken for loss on drying determination. The test result should be ≤2%; otherwise, it was dried at 60 ± 5°C until the loss on drying test result was ≤2%. It was then milled using an FHM-67 sizer and passed through a 40-mesh sieve, with parameters as shown in Table 2.

**Table 2 HPMCAS-HG milling parameters**

| Item | Set reference value |
|---|---|
| Rotation speed | 5500 ± 500 rpm |
| Sieve size | 0.8 mm |
| Mill configuration | Hammer forward |
| Alarm temperature | 50°C |

Additionally, the present invention conducted equilibrium solubility experiments of the monohydrate crystalline API, amorphous API, and hot-melt extrudate dispersion intermediate in buffer media at different pH conditions. The data are shown in Table 3 and FIG. 1.

**Table 3. Summary of equilibrium solubility (µg/ml) of monohydrate crystalline API, amorphous API, and hot-melt extrudate dispersion intermediate**

| Compound of formula (I) (monohydrate crystalline form) | | | Compound of formula (I) (amorphous API) | | | Compound of formula (I) (hot-melt extrusion) | | |
|---|---|---|---|---|---|---|---|---|
| pH | 1-h solubility (µg/ml) | 22-h solubility (µg/ml) | pH | 1-h solubility (µg/ml) | 22-h solubility (µg/ml) | pH | 1-h solubility (µg/ml) | 22-h solubility (µg/ml) |
| 1.2 | 30.29 | 27.16 | 1.2 | 50.93 | 48.62 | 1.2 | 139.30 | 134.09 |
| 2.0 | 3.57 | 3.22 | 2.0 | 4.52 | 4.45 | 2.0 | 30.24 | 30.40 |
| 3.0 | 1.07 | 0.97 | 3.0 | 2.14 | 2.14 | 3.0 | 12.85 | 11.89 |
| 4.0 | 0.68 | 0.60 | 4.0 | 0.76 | 0.74 | 4.0 | 4.99 | 4.13 |
| 4.5 | 0.60 | 0.59 | 4.5 | 0.65 | 0.57 | 4.5 | 4.42 | 4.15 |
| 5.0 | 0.47 | 0.51 | 5.0 | 0.51 | 0.42 | 5.0 | 5.94 | 4.43 |
| 6.0 | 0.50 | 0.45 | 6.0 | 0.46 | 0.44 | 6.0 | 9.53 | 5.95 |
| 6.8 | 0.44 | 0.42 | 6.8 | 0.46 | 0.45 | 6.8 | 10.27 | 10.82 |
| 7.5 | 0.42 | 0.43 | 7.5 | 0.54 | 0.48 | 7.5 | 8.50 | 8.32 |

As can be seen from Table 3 and FIG. 1, (1) the hot-melt extrudate dispersion intermediate can significantly improve the solubility of API in different media; (2) the amorphous form and monohydrate without preparation via the hot-melt extrusion process exhibit essentially identical solubility, showing no definitive improvement; this indicates that the original amorphous form alone does not possess the inherent ability to enhance solubility, and it is speculated that the amorphous API without preparation via hot-melt extrusion would rapidly precipitate out as the more stable monohydrate crystalline form in aqueous media; (3) the hot-melt extrudate dispersion intermediate can inhibit the rapid precipitation of API into the more stable monohydrate crystalline form.

The hot-melt extrudate dispersion intermediate of the present invention can be milled to a selected particle size distribution. In addition to this advantage, the formulation obtained by the melt extrusion process also possesses the following advantages: ① the hot-melt extrusion process can effectively combine the drug with a polymer through thermal and mechanical energy to form an amorphous state, which can maintain long-term drug stability, enhance drug solubility, and further improve the bioavailability of the poorly soluble drug in vivo; ② the application of hot-melt extrusion technology requires no solvent, thereby reducing solvent costs and environmental pressure; and ③ compared to spray drying, the equipment used in hot-melt extrusion occupies less space, especially at the scale-up production level.

### 2. Investigation of secondary milling process

The present invention investigated the effect of particle size on the dissolution curve of hot-melt extrudate dispersion intermediate (HMEDI). The hot-melt extrudate dispersion intermediate (HMEDI) was milled and sieved to separate samples with three particle size distributions (PSD): <100 µm, 100 to 250 µm and >250 µm. HMEDI granules with particle size <100 µm and particle size 100-250 µm achieved complete dissolution; larger granules with particle size >250 µm did not dissolve smoothly and tended to agglomerate, forming a gel and resulting in a lower concentration of free compound of formula (I) under SGF and SIF conditions. Based on these results, a PSD with suitable dissolution characteristics was selected for further development, and tablet mixture adjuvants with similar PSD were used to support suitable mixing uniformity.

Reference values: D₉₀ less than 300 µm (as a more stringent requirement, D₉₀ target is less than 50 mesh), D₅₀ less than 120 µm (as a more stringent requirement, D₅₀ target is less than 120 mesh or D₄₀ is less than 140 mesh).

The hot-melt extrudate dispersion intermediate (HMEDI) of the desired PSD was produced by milling and sieving and directly using a hammer mill (with appropriate hammering speed and sieve size). Feasibility study on tableting using these batches of hot-melt extrudate dispersion intermediates (HMEDIs) was conducted.

The secondary milling process imparts better flowability and millability to the material during secondary sizing, along with improved dissolution release behavior.

The material after passing through the 40-mesh sieve was tested for particle size distribution, and the results are as shown in Table 4.

**Table 4. Particle size distribution of HPMCAS-HG after milling and passing through a 40-mesh sieve.**

| Sieve aperture (mesh/micrometer) | Sieve tare weight, A | Gross weight with granules, B | Calculated value (B-A) | Percentage |
|---|---|---|---|---|
| >40 mesh/380 µm | / | / | / | 0 |
| 40 mesh/380 µm to 50 mesh/270 µm | 318.8 g | 325.0 g | 6.2 g | 10.9% |
| 50 mesh/270 µm to 60 mesh/250 µm | 322.8 g | 329.8 g | 7.0 g | 12.3% |
| <60 mesh/250 µm | 359.2 g | 402.8 g | 43.6 g | 76.8% |

The particle size distribution indicates that under these milling conditions, D(v,0.9) ≈ 270 µm, which meets the requirement that the particle size distribution of HPMCAS-HG, D(v,0.9), is less than 250-300 µm. This milling process can effectively control the particle size distribution range of HPMCAS-HG.

HMEDI milling: bulk density and particle size distribution of milled powder intermediate

The particle size of HMEDI plays an important role in the downstream processes of the final solid dosage form. Therefore, a study on the milling process was conducted to investigate the effects of milling speed and sieve size on the particle size distribution of the milled extrudate. The hot-melt extrudate of 60% of the compound of formula (I) was fed from the extruder onto cooling rollers to divide the extrudate into free-flowing flakes. Under various milling conditions, about 150 g of the HME flake sample of the compound of formula (I) was fed into a hammer mill equipped with forward-facing hammers for milling to achieve a significant reduction in particle size. The milling speed and sieve size were varied until the particle size distribution was consistently reduced to approximately D(v,0.9) < 300 µm and D(v,0.5) < 120 µm (as shown in Table 5, Test Nos. 1-3).

**Table 5. In-process particle size testing results for milled hot-melt extrudate containing 60% of the compound of formula (I)**

| Test No. | Milling speed (RPM) | Sieve size (mesh) | D₁₀ (µm) | D₅₀ (µm) | D₉₀ (µm) | Morphology |
|---|---|---|---|---|---|---|
| 1 | 5500 | 27 | 25 | 117 | 262 | Unimodal |
| 2 | 3000 | 20 | 39 | 154 | 330 | Unimodal |
| 3 | 5500 | 20 | 29 | 115 | 255 | Unimodal |
| 4 | 5500 | 20 | 24 | 96 | 222 | Unimodal |

The data showed that particle size decreased with increasing milling speed. Although particle size showed no significant difference with changes in sieve size, retention of larger granules was observed with the 27-mesh sieve, so the 27-mesh sieve was rejected. Based on the results of the milling study, the final selected milling parameters are shown in Table 6. The HME milling process was scaled up using 4 kg of the flake extrudate of the compound of formula (I) to prepare HMEDI. XRPD and DSC analysis showed that the HMEDI batch was in non-crystalline state. The particle size distribution was unimodal, with D(v,0.9) approximately < 222 µm (as shown in Table 5, Test No. 4). The bulk density and tap density were found to be 0.55 g/mL and 0.71 g/mL, respectively. The bulk density and particle size distribution were controlled within ranges suitable for uniform mixing with tableting adjuvants.

**Table 6 Final milling parameters of hot-melt extrudate (HMEDI) of 60% of compound of formula (I)**

| Item | Set reference value |
|---|---|
| Milling speed | 5500 rpm |
| Sieve size | 20 mesh |
| Mill configuration | Hammer forward |

The 20-mesh sieve is included with the secondary sizer.

In the production of pilot-scale batches with various strengths (i.e., small-scale trials of the product before large-scale mass production) in the present invention, the material after hot-melt extrusion and cooling was taken and subjected to primary milling in a milling machine equipped with a 2.0 mm-aperture sieve at a milling frequency of 40±5 Hz. After milling, a sizer equipped with a 0.8 mm-aperture sieve was used for secondary sizing at a milling speed of 5500±500 rpm. The granules after secondary sizing were then passed through a 40-mesh sieve to remove large granules. The secondary sizing parameters are shown in Table 7.

**Table 7. Secondary sizing parameters for pilot-scale batches**

| Item | Set reference value |
|---|---|
| Rotation speed | 5500 ± 500 rpm |
| Sieve size | 0.8 mm |
| Mill configuration | Hammer forward |
| Alarm temperature | 50°C |

In the production of pilot-scale batches (Batch Nos. R4020001 to R4020009), approximately 50 g of the material after secondary sizing was taken from each batch for particle size distribution testing. 40-mesh, 50-mesh, 60-mesh, 70-mesh, and 140-mesh sieves were used. The results showed that the particle size distribution after secondary sizing for all pilot-scale batches was less than 270 µm for D(v, 0.9), and approximately less than 120 µm for D(v, 0.5), meeting the control requirements of D(v, 0.9) being approximately less than 300 µm and D(v, 0.5) being approximately less than 120 µm.

The bulk density and tap density of the pilot-scale batches were controlled within ranges suitable for uniform mixing with tableting adjuvants, at 0.56-0.65 and 0.70-0.73, respectively.

### 3. Selection of adjuvants in the pharmaceutical composition - optimization of direct compression of final blend granules

Increasing the amount of friable material in the formulation can improve the compression profile, tablet appearance, and surface strength, thereby improving friability performance. The early-stage formulations were compressed using a single-punch tablet press. The results showed that increasing the ratio of mannitol M100 to microcrystalline cellulose PH102 improved the compressibility of the final blend granules. However, even with increased compression pressure and tablet hardness, disintegration was still too rapid (<1 min). The disintegration time was inversely proportional to the proportion of croscarmellose sodium used in the formulation. With 0.5% w/w croscarmellose sodium, the final blend granules showed an approximate linear correlation among increasing compression pressure, increasing tablet hardness, and increasing disintegration time. To optimize the formulations, the amount of lubricant magnesium stearate 2257 was increased from 0.5% to 1.5%, which reduced the ejection force below 300 N. The final optimized formulations are shown in Table 8.

**Table 8. Content of each component in the optimized formulations**

| Component | Theoretical amount (mg/tablet) / Batch size 1000 tablets | | |
|---|---|---|---|
| | 25 mg strength | 50 mg strength | 75 mg strength |
| Compound of formula (I)* | 25.00 | 50.00 | 75.00 |
| Hypromellose acetate succinate AS-HG (HPMCAS-HG) | 16.67 | 33.34 | 50.01 |
| Colloidal silicon dioxide M5P | 1.50 | 3.00 | 4.50 |
| Microcrystalline cellulose PH102 | 21.93 | 43.86 | 65.79 |
| Mannitol M100 | 32.90 | 65.80 | 98.70 |
| Croscarmellose sodium | 0.50 | 1.00 | 1.50 |
| Magnesium stearate 2257 | 1.50 | 3.00 | 4.50 |
| Total weight | 100.00 | 200.00 | 300.00 |

Small-scale tablets obtained by compression on a single-punch tablet press using the optimized formulations had suitable hardness and disintegration results. 25 mg tablets (Batch No. T7-127-92) and 100 mg tablets (Batch No. T7-127-93) had good tablet characteristics. The results are as shown in Table 9.

**Table 9 Average compressive force, hardness, and disintegration time for tablets of 25 mg and 100 mg strengths**

| Tablet | Batch No. | Compressive force (kN) | Hardness (kP) | Disintegration (min) |
|---|---|---|---|---|
| 25 mg strength | T7-127-92 | 7 | 7 | 3.3 |
| 100 mg strength | T7-127-93 | 13 | 15 | 3.0 |

The formulation composition during the process scale-up is shown in Table 10.

**Table 10 Formulation composition during the process scale-up**

| Component | Theoretical amount (kg) /10000 tablets | | | Excessive addition | Function |
|---|---|---|---|---|---|
| | 25 mg strength | 50 mg strength | 75 mg strength | | |
| Compound of formula (I)* | 0.250 | 0.500 | 0.750 | None | Active ingredient |
| Hypromellose acetate succinate AS-HG (HPMCAS-HG) | 0.1667 | 0.3334 | 0.5001 | None | Solid dispersion carrier |
| Colloidal silicon dioxide M5P | 0.015 | 0.030 | 0.045 | None | Glidant |
| Microcrystalline cellulose PH102 | 0.2193 | 0.4386 | 0.6579 | None | Water-insoluble filler |
| Mannitol M100 | 0.329 | 0.658 | 0.987 | None | Insoluble filler |
| Croscarmellose sodium | 0.005 | 0.010 | 0.015 | None | Disintegrant |
| Magnesium stearate 2257 | 0.015 | 0.030 | 0.045 | None | Lubricant |
| Total weight | 1.0 | 2.0 | 3.0 | / | |
| Solvent used and ultimately removed in the process | None | | | | |

| | | | | | |
|---|---|---|---|---|---|
| Note: * as anhydrous free base equivalent. The actual amount of the compound of formula (I) will be adjusted according to the content of each active pharmaceutical ingredient batch. | | | | | |

### 4. Impurity profile and stability under long-term storage conditions at 25°C for hot-melt extrudate dispersion intermediate (HMEDI)

This study found that since the hot-melt extrusion process involves melting and mixing the active pharmaceutical ingredient (i.e., the compound of formula (I)) with HPMCAS-HG polymer at elevated temperature to form a non-crystalline extrudate, the validated current method for related substances in the formulation was used to evaluate the hot-melt extrudate dispersion intermediate (HMEDI). This was done to investigate any differences in the purity of the API in HMEDI compared to the initial purity of the API used in each batch. No new degradation products were found in all batches of hot-melt extrudate dispersion intermediate (HMEDI). The purity and impurity profiles remained essentially unchanged compared to the corresponding API batches used. This indicates that no significant degradation occurred during the hot-melt extrusion process and no chemical interaction with the polymer auxiliary material took place.

The stability study results of the three strengths of the inventive tablets from Table 10, stored at 40°C for 6 months and at 25°C for 18 months, show that the HMEDI purity and content of the batch remained essentially unchanged, with no significant degradation trend; the moisture content did not undergo any significant change; XRPD results indicated an amorphous state, suggesting no change in crystalline form during storage; and the DSC profile remained unchanged. The above results indicate that the formulations are stable.

### 5. Tablet manufacturing process flow

### ① Pre-treatment of raw and auxiliary materials

Before tablet production, the crystalline form and particle size distribution of the compound of formula (I) were determined. The reference crystalline form of the compound of formula (I) was the free base monohydrate, and the reference particle size distribution should satisfy D₅₀ ≤ 105.77 µm and D₉₀ ≤ 229.88 µm.

Hypromellose acetate succinate AS-HG was taken for loss on drying determination. The test result should be ≤ 2%; otherwise, it was dried at 60 ± 5°C until the moisture test result was ≤ 2%. Milling was performed using an FHM-67 sizer equipped with a 0.8 mm-aperture sieve at a milling speed of 5500 ± 500 rpm. The milled material was passed through a 40-mesh sieve.

Colloidal silicon dioxide M5P, microcrystalline cellulose PH102, mannitol M100, and croscarmellose sodium were passed through a 30-mesh sieve, magnesium stearate 2257 was passed through a 40-mesh sieve, and the compound of formula (I) was passed through a 30-mesh sieve. In this way, possible agglomerates in the materials during storage were removed.

### ② Pre-mixing

The formulation amounts of the compound of formula (I) and hypromellose acetate succinate AS-HG were weighed and added to the 5L hopper of an HLS50 laboratory mixer. Mixing was performed under the following set conditions: mixing time 15 min and mixing speed 20 rpm.

### ③ Hot-melt extrusion granulation

Hot-melt extrusion: the extrusion parameters were set as shown in Table 11. The uniformly pre-mixed raw and auxiliary materials were taken and added manually into the hot-melt extruder. By adjusting the feeding speed and the extrusion screw speed, the feeding speed can be matched with the discharge speed.

**Table 11 Extrusion parameters for hot-melt extrusion granulation**

| Heating section (°C) | | | | | Die temperature, °C | Extruder speed, rpm | Melt pressure, bar | Extruder energy consumption, % | Cooling belt speed, m/min |
|---|---|---|---|---|---|---|---|---|---|
| Zone 1 temperature | Zone 2 temperature | Zone 3 temperature | Zones 4 to 10 temperature | Zone 11 temperature | | | | | |
| 90 ± 20 | 130 ± 20 | 160 ± 5 | 160 ± 5 | 160 ± 20 | 160 ± 10 | 100 ± 25 | ≤ 150 | ≤ 80 | 1-10 |

Milling: the material after hot-melt extrusion and cooling was taken and subjected to primary milling in a milling machine equipped with a 2.0 mm-aperture sieve at a milling frequency of 40±5 Hz. After milling, a sizer equipped with a 0.8 mm-aperture sieve was used for secondary sizing at a milling speed of 5500±500 rpm. The granules after secondary sizing were passed through a 40-mesh sieve.

### ④ Final blending

### Final blending 1

Based on the weight after milling and calculation according to the formulation, colloidal silicon dioxide M5P and part of microcrystalline cellulose PH102 were first manually mixed and then added to a 10L hopper. Then, the granules after hot-melt granulation were added to the hopper along with mannitol M100, croscarmellose sodium and the remaining microcrystalline cellulose PH102. Mixing was performed under the following set conditions: mixing time 15 min and mixing speed 20 rpm.

### Final blending 2

Magnesium stearate 2257 after passing through the 40-mesh sieve was added to the mixing hopper. Mixing was performed under the following set conditions: mixing time 4 min and mixing speed 20 rpm. The loss on drying of the granules after final blending should be ≤ 5.0%, and the content of the compound of formula (I) should be 90.0% to 110.0% of the labeled content.

### ⑤ Tableting

A high-speed tablet press was used for compression. The individual tablet weight variation of the tablets of the present invention was controlled to within ±7.5%, the average tablet weight variation to within ±5.0%, the hardness range to 9.0-12.0 kp, the friability to ≤0.5%, and the disintegration time limit requirement to complete disintegration within 15 min.

### ⑥ Bottling

30 tablets were counted using a tablet counting plate, and filled into a high-density polyethylene bottle for oral solid medicine. The bottle was sealed by screwing on a safety cap followed by aluminum foil sealing with a sealing machine.

The sealed sample bottle was inspected, and the sealing integrity was required to comply with the specified standards.

The hot-melt extrudate dispersion intermediate (HMEDI) was designed to maintain API in the non-crystalline form in an aqueous environment, but to release API rapidly in the intestine for systemic absorption. Four tablet strengths [25 mg, 50 mg, 75 mg and 100 mg] were studied. Four batches of final blend material with appropriate properties were compressed via different punch and die sets. The formulations are shown in Table 12, with component amounts scaled proportionally.

**Table 12 Composition of the final blend for the tablets of the present invention with 25mg, 50mg, 75mg and 100mg strengths**

| Component | Unit composition | | | |
|---|---|---|---|---|
| | Weight % | Formulation amounts of materials per tablet for each strength | | |
| | (w/w %) | 25 mg | 50 mg | 75 mg |
| HMEDI (600 mg/g) | 40.67 | 40.67 | 83.34 | 125.01 |
| Mannitol M100 | 32.90 | 32.90 | 65.80 | 98.70 |
| Microcrystalline cellulose PH102 | 21.93 | 21.93 | 43.86 | 65.79 |
| Croscarmellose sodium | 0.5 | 0.5 | 1.00 | 1.50 |
| Colloidal silicon dioxide M5P | 1.50 | 1.50 | 3.00 | 4.50 |
| Magnesium stearate 2257 | 1.50 | 1.50 | 3.00 | 4.50 |
| Total: | 100.00 | 100.00 | 200.00 | 300.00 |

### 6. Tablet testing

### 6.1. Dissolution

### 6.1.1. Dissolution conditions

Test objects: Pilot-scale batches (Batch Nos. R4020001, R4020002, R4020007). The preparation process was in accordance with "5. Tablet manufacturing process flow". Dissolution medium: as shown in Table 13. Method: General Chapter 0931 of Part IV of the Chinese Pharmacopoeia, Method II, Paddle Method. Rotation speed: 75 revolutions per minute. Sampling points: Samples were separately taken at the designated time points. The subsequent filtrate was used as the test solution, without replenishing with an equal volume of dissolution medium at the same temperature.

**Table 13 Conditions for dissolution curve study**

| Method | Dissolution medium | Rotation speed (rpm) | Sampling time point (min) |
|---|---|---|---|
| 1 | 0.01 mol/L hydrochloric acid solution containing 0.5% SLS | 75 | 5, 10, 15, 30, 45, 60 |
| 2 | pH 3.0 citrate buffer containing 0.5% SLS | 75 | 5, 10, 15, 30, 45, 60 |
| 3 | pH 4.5 acetate buffer containing 0.5% SLS | 75 | 5, 10, 15, 30, 45, 60 |
| 4 | pH 6.8 phosphate buffer containing 0.5% SLS | 75 | 5, 10, 15, 30, 45, 60 |

Among these, the 0.01 mol/L hydrochloric acid solution containing 0.5% SLS (Method 1) was used as the standard dissolution medium.

### 6.1.2. Measurement method

A Sigma Ascentis Express C₁₈ 2.7 µm, 4.6 x 100 mm column or equivalent was used, with water-acetonitrile = 50:50 (containing 0.1% formic acid (chromatographic grade)) as mobile phase, flow rate: 0.75 ml/min; column temperature: 40°C; detection wavelength: 240 nm. Approximately 15 mg of the reference standard of the compound of formula (I) was accurately weighed, placed into a 100 mL volumetric flask, dissolved in 20 ml of acetonitrile by ultrasonication, and then diluted to volume with the dissolution medium to obtain the reference standard solution. Accurately measured aliquots (3 µl each) of the reference standard solution and the test sample solution were separately injected into the liquid chromatograph. The chromatograms were recorded, and the cumulative dissolution percentage at each time point was calculated using the peak areas by the external standard method, followed by the plotting of the dissolution profile.

### 6.1.3. Results

The dissolution results of the pilot-scale batch samples for the 25 mg (Batch No. R4020001), 50 mg (Batch No. R4020002), and 75 mg (Batch No. R4020007) strength tablets in four dissolution media are shown in Tables 14, 15, 16, and 17.

**Table 14 Summary of dissolution results of the tablets of the present invention in 0.01 mol/L hydrochloric acid solution containing 0.5% SLS**

| Time (min) | Tablets of this invention | | | | | |
|---|---|---|---|---|---|---|
| | 25 mg | | 50 mg | | 75 mg | |
| | Batch No. R4020001 | | Batch No. R4020002 | | Batch No. R4020007 | |
| | AVG (%) | RSD | AVG (%) | RSD | AVG (%) | RSD |
| 5 | 23.7 | 13.86 | 18.6 | 27.66 | 23.8 | 27.51 |
| 10 | 46.5 | 5.93 | 41.3 | 11.00 | 43.5 | 13.63 |
| 15 | 61.8 | 3.38 | 57.3 | 6.20 | 58.3 | 9.17 |
| 30 | 86.1 | 1.36 | 83.6 | 1.80 | 83.5 | 3.57 |
| 45 | 96.1 | 1.36 | 94.6 | 1.09 | 95.0 | 2.88 |
| 60 | 99.1 | 1.12 | 99.6 | 1.43 | 100.9 | 0.43 |

**Table 15 Summary of dissolution results of the tablets of the present invention in pH 3.0 citrate buffer containing 0.5% SLS**

| Time (min) | Tablets of this invention | | | | | |
|---|---|---|---|---|---|---|
| | 25 mg | | 50 mg | | 75 mg | |
| | Batch No. R4020001 | | Batch No. R4020002 | | Batch No. R4020007 | |
| | AVG (%) | RSD | AVG (%) | RSD | AVG (%) | RSD |
| 5 | 27.8 | 6.36 | 24.8 | 15.13 | 26.4 | 13.83 |
| 10 | 50.8 | 2.62 | 50.1 | 4.74 | 48.1 | 3.77 |
| 15 | 66.7 | 1.78 | 67.7 | 2.11 | 63.6 | 1.84 |
| 30 | 91.1 | 1.76 | 93.0 | 1.11 | 88.2 | 1.10 |
| 45 | 100.0 | 2.19 | 101.8 | 1.76 | 98.0 | 1.57 |
| 60 | 103.2 | 2.50 | 104.5 | 1.84 | 101.4 | 1.89 |

**Table 16 Summary of dissolution results of the tablets of the present invention in pH 4.5 acetate buffer containing 0.5% SLS**

| Time (min) | Tablets of this invention | | | | | |
|---|---|---|---|---|---|---|
| | 25 mg | | 50 mg | | 75 mg | |
| | Batch No. R4020001 | | Batch No. R4020002 | | Batch No. R4020007 | |
| | AVG (%) | RSD | AVG (%) | RSD | AVG (%) | RSD |
| 5 | 33.5 | 5.95 | 29.8 | 8.27 | 27.5 | 11.16 |
| 10 | 54.9 | 2.96 | 54.2 | 3.06 | 48.3 | 4.68 |
| 15 | 69.1 | 2.26 | 68.7 | 2.12 | 63.0 | 3.29 |
| 30 | 89.9 | 1.26 | 89.6 | 1.54 | 85.3 | 2.88 |
| 45 | 97.8 | 1.15 | 97.4 | 1.55 | 95.2 | 1.76 |
| 60 | 101.0 | 1.39 | 100.3 | 1.45 | 99.4 | 2.08 |

**Table 17 Summary of dissolution results of the tablets of the present invention in pH 6.8 phosphate buffer containing 0.5% SLS**

| | Tablets of this invention | | | | | |
|---|---|---|---|---|---|---|
| Time (min) | 25 mg | | 50 mg | | 75 mg | |
| | Batch No. R4020001 | | Batch No. R4020002 | | Batch No. R4020007 | |
| | AVG (%) | RSD | AVG (%) | RSD | AVG (%) | RSD |
| 5 | 39.8 | 8.72 | 35.0 | 6.41 | 40.0 | 6.67 |
| 10 | 67.6 | 2.06 | 64.7 | 1.42 | 66.2 | 3.40 |
| 15 | 81.4 | 1.31 | 79.7 | 1.38 | 80.9 | 2.87 |
| 30 | 95.8 | 1.55 | 95.6 | 1.55 | 98.2 | 3.05 |
| 45 | 100.0 | 2.08 | 99.7 | 1.44 | 101.8 | 3.14 |
| 60 | 101.5 | 1.51 | 101.0 | 1.52 | 104.4 | 2.78 |

### 6.1.4. Conclusion

Dissolution testing of all three tablet strengths was conducted under standard non-sink conditions. The release profiles were similar across strengths, with more than 80% release at 30 min and complete release achieved within about 45 to 60 min.

The dissolution behaviors were essentially consistent across the four different media, and the resulting dissolution profiles exhibited small batch-to-batch variations. For the three tablet batches, the relative standard deviation (RSD) of dissolution values at all corresponding time points across the four dissolution profiles was less than 10%, with similar dissolution trends and endpoints.

The hot-melt extrudate dispersion intermediate (HMEDI) was designed to maintain API in the non-crystalline form in an aqueous environment, but to release API rapidly in the intestine for systemic absorption.

### 6.2 Pharmacokinetic Parameters

Tablets with Batch No. R4020002 and the earlier development batch No. G-18-014A were manufactured as described in "5. Tablet manufacturing process flow," and their corresponding pharmacokinetic parameters are shown in Table 18.

**Table 18 Plasma drug concentration data and pharmacokinetic parameters in beagle dogs following a single oral administration of tablets with Batch Nos. R4020002 and G-18-014A (mean results of three beagle dogs).**

| Batch No. | R4020002 | G-18-014A |
|---|---|---|
| Sampling time | Concentration (ng/mL) | |
| 0 | 0 | 0 |
| 0.25 | 13.9 ± 14.2 | 31.6 ± 37.6 |
| 0.5h | 421.3 ± 170.5 | 293.8 ± 235.3 |
| 1h | 1470.0 ± 350.3 | 1222.3 ± 502.3 |
| 2h | 2856.7 ± 532.9 | 2083.3 ± 1486.5 |
| 4h | 2870.0 ± 593 | 2226.7 ± 1920.0 |
| 8h | 605.0 ± 201.2 | 738.3 ± 167.2 |
| 24h | 206.3 ± 235.2 | 156.3 ± 120.7 |

| | PK parameters | |
|---|---|---|
| Cₘₐₓ (ng/mL) | 3016.7 ± 516.2 | 2303.3 ± 1864.0 |
| Tₘₐₓ (h) | 2.67 ± 1.15 | 2.67 ± 1.15 |
| T_{1/2} (h) | 4.08 ± 0.87 | 6.65 ± 4.08 |
| AUC₀-ₜ (ng*h/mL) | 21859.1 ± 1659.5 | 19474.0 ± 10154.3 |
| AUC₀-_{inf} (ng*h/mL) | 21400.1 ± 1165.5 | 15706.2 ± 6126.9 |
| AUMC₀-ₜ (ng·h²/mL) | 132195 ± 42925 | 123027 ± 53040 |
| AUMC₀-_{inf} (ng·h²/mL) | 121187 ± 10083 | 169519 ± 138821 |

The tablets of the present invention exhibit similar in vitro release behavior, indicating that the dissolution conditions in the quality standard can adequately reflect in vitro-in vivo correlation.

The embodiments of the present invention have been described in detail above. Specific examples are used in this document to explain the principles and implementations of the present invention. The descriptions of the above embodiments are provided solely to aid in understanding the method and core concept of the present invention. Meanwhile, any modifications or variations made by those skilled in the art based on the concept of the present invention, within the specific implementations and application scope thereof, shall fall within the scope of protection of the present invention. In summary, the content of this specification shall not be construed as limitations to the present invention.

## Claims

1. A pharmaceutical composition containing a heterocyclic compound, **characterized in that** the pharmaceutical composition comprises the following components:
(a) a compound of formula (I) 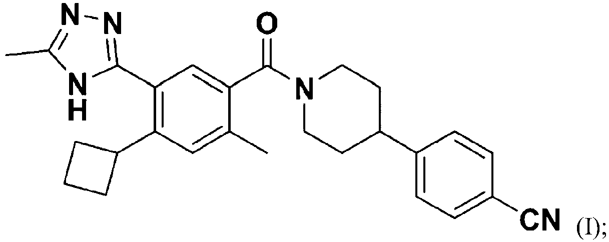 and
(b) hypromellose acetate succinate or copovidone;
wherein component (a) and component (b) are mixed and subjected to hot-melt extrusion or spray drying;
wherein the weight ratio of component (b) to component (a) is from 0.4 to 0.8.

2. The pharmaceutical composition according to claim 1, **characterized in that** components (a) and (b) are mixed and subjected to hot-melt extrusion or spray drying at a temperature ranging from 145°C to 200°C;
preferably, components (a) and (b) are mixed and subjected to hot-melt extrusion or spray drying at a temperature ranging from 155°C to 180°C;
preferably, components (a) and (b) are mixed and subjected to hot-melt extrusion or spray drying at a temperature ranging from 155°C to 165°C;
more preferably, the hypromellose acetate succinate is hypromellose acetate succinate of HG grade;
more preferably, the weight ratio of component (b) to component (a) is from 0.5 to 0.7;
more preferably, the weight ratio of component (b) to component (a) is about 0.66.

3. The pharmaceutical composition according to claim 1, **characterized in that** based on the total weight of the pharmaceutical composition, the pharmaceutical composition further comprises from 50% to 70% by weight, for example from 55% to 65% by weight, of one or more pharmaceutically acceptable adjuvants, wherein the pharmaceutically acceptable adjuvants are selected from one or more of the following: water-soluble filler, water-insoluble filler, disintegrant, glidant, and lubricant;
preferably, the water-soluble filler is mannitol, for example mannitol M100;
preferably, the water-insoluble filler is microcrystalline cellulose, for example microcrystalline cellulose PH102;
preferably, the disintegrant is croscarmellose sodium;
preferably, the glidant is colloidal silicon dioxide, for example colloidal silicon dioxide M5P;
preferably, the lubricant is magnesium stearate, for example magnesium stearate 2257;
more preferably, based on the total weight of the pharmaceutical composition, the pharmaceutical composition further comprises from 30% to 35% by weight of a water-soluble filler, for example about 33% by weight;
more preferably, based on the total weight of the pharmaceutical composition, the pharmaceutical composition further comprises from 20% to 25% by weight of a water-insoluble filler, for example about 22% by weight;
more preferably, based on the total weight of the pharmaceutical composition, the pharmaceutical composition further comprises from 0.2% to 1% by weight of a disintegrant, for example about 0.5% by weight;
more preferably, based on the total weight of the pharmaceutical composition, the pharmaceutical composition further comprises from 1% to 3% by weight of a glidant, for example about 1.5% by weight;
more preferably, based on the total weight of the pharmaceutical composition, the pharmaceutical composition further comprises from 1% to 3% by weight of a lubricant, for example about 1.5% by weight.

4. The pharmaceutical composition according to any one of claims 1 to 3, **characterized in that** the dosage form of the pharmaceutical composition is a tablet, granule, or capsule;
preferably, the pharmaceutical composition further comprises one or more drugs and/or extracts;
more preferably, the drug is an anti-fatty liver drug, an anticancer drug, and/or an anti-acne drug.

5. A method for preparing the pharmaceutical composition according to any one of claims 1 to 4, **characterized in that** the method comprises the following steps:
(1) mixing sieved component (a) with dried, milled, and sieved component (b) to obtain a mixture;
(2) extruding the mixture via hot-melt extrusion at a hot-melt extrusion temperature of 145°C to 200°C to form an extrudate, or dissolving the mixture in an organic solvent, such as ethanol, dichloromethane, acetonitrile, etc., for spray drying;
(3) cooling the extrudate or spray-dried material; and
(4) subjecting the cooled extrudate or spray-dried material to milling, sizing, and sieving to obtain granules of the pharmaceutical composition.

6. The method according to claim 5, **characterized in that** the method further comprises the step of:
(5) mixing and drying the granules with one or more sieved pharmaceutically acceptable adjuvants, and processing into a tablet or capsule;
preferably, the mixing comprises first performing a first mix of the glidant and a portion of the water-insoluble filler; then adding the granules, the water-soluble filler, the disintegrant, and the remaining water-insoluble filler for a second mix; and finally adding the lubricant for mixing;
more preferably, the component (b) is hypromellose acetate succinate of HG grade;
more preferably, the water-soluble filler is mannitol, for example mannitol M100;
more preferably, the water-insoluble filler is microcrystalline cellulose, for example microcrystalline cellulose PH102;
more preferably, the disintegrant is croscarmellose sodium;
more preferably, the glidant is colloidal silicon dioxide, for example colloidal silicon dioxide M5P;
more preferably, the lubricant is magnesium stearate, for example magnesium stearate 2257;
further preferably, **characterized in that** a twin-screw hot-melt extrusion apparatus is used to extrude the mixture of components (a) and (b), wherein the screw diameter of the twin-screw hot-melt extrusion is between 8 mm and 50 mm, and the extrusion speed is between 10 rpm and 300 rpm;
further preferably, the screw diameter of the twin-screw hot-melt extrusion is 18 mm;
further preferably, the extrusion speed is from 75 rpm to 125 rpm, for example about 100 rpm.

7. The method according to claim 5 or 6, **characterized in that** steps (1) to (5) of the method comprise any one or more of the following items [1] to [28]:
[1] the loss on drying determination in step (1) shows a result of not more than 2%;
[2] the drying temperature in step (1) is 55°C to 65°C, for example about 60°C;
[3] the milling speed in step (1) is 5000 rpm to 6000 rpm, for example about 5500 rpm;
[4] the sieve in step (1) is a 30-mesh sieve or a 40-mesh sieve;
[5] the mixing time in step (1) is 10 min to 20 min, for example about 15 min;
[6] the mixing speed in step (1) is 15 rpm to 25 rpm, for example about 20 rpm;
[7] the temperature of zone 1 of the extruder in step (2) is 70°C to 110°C, for example about 90°C;
[8] the temperature of zone 2 of the extruder in step (2) is 110°C to 150°C, for example about 130°C;
[9] the temperature of zone 3 of the extruder in step (2) is 155°C to 165°C, for example about 160°C;
[10] the temperatures of zones 4-10 of the extruder in step (2) are 155°C to 165°C, for example about 160°C;
[11] the temperature of zone 11 of the extruder in step (2) is 140°C to 180°C, for example about 160°C;
[12] the die temperature of the extruder in step (2) is 150°C to 170°C, for example about 160°C;
[13] the melt pressure of the extruder in step (2) is not more than 150 bar;
[14] the load pressure of the extruder in step (2) is not more than 80%, for example 25% to 65%;
[15] the cooling belt speed of the extruder in step (2) is 1 m/min to 10 m/min;
[16] the processing rate of the extruder in step (2) is 0.1 kg/h to 10 kg/h;
[17] the sieve aperture for milling in step (4) is about 2 mm;
[18] the milling frequency in step (4) is 35 Hz to 45 Hz, for example about 40 Hz;
[19] the milling speed in step (4) is 5000 rpm to 6000 rpm, for example about 5500 rpm;
[20] the rotation speed for sizing in step (4) is 5000 rpm to 6000 rpm, for example about 5500 rpm;
[21] the sieve aperture for sizing in step (4) is about 0.8 mm;
[22] the sieve in step (4) is a 40-mesh sieve;
[23] the particle size of the granules in step (4) is not more than 250 µm, for example 100 µm to 250 µm;
[24] the time for the first mix in step (5) is 10 min to 20 min, for example about 15 min;
[25] the speed for the first mix in step (5) is 15 rpm to 25 rpm, for example about 20 rpm;
[26] the time for the second mix in step (5) is 2 min to 6 min, for example about 4 min;
[27] the speed for the second mix in step (5) is 15 rpm to 25 rpm, for example about 20 rpm;
[28] the loss on drying determination in step (5) shows a result of not more than 5%.

8. The pharmaceutical composition according to any one of claims 1 to 4, wherein the compound of formula (I) is in an amorphous solid form;
preferably, the amorphous solid form, when analyzed thermally using differential scanning calorimetry (DSC), exhibits an endothermic peak at approximately 98°C;
preferably, the amorphous solid form, when analyzed thermally using modulated differential scanning calorimetry (MDSC), has a glass transition temperature of about 113°C;
preferably, the amorphous solid form has an x-ray powder diffraction (XRPD) pattern substantially as shown in FIG. 2.

9. The pharmaceutical composition according to any one of claim 8, wherein the amorphous solid form is prepared as follows:
a hot-melt extrudate dispersion intermediate is prepared by using specific adjuvants through a hot-melt extrusion process or a spray drying process, which process transforms the compound of formula (I) from a crystalline form into a stable, non-crystalline amorphous form.

10. Use of the pharmaceutical composition according to any one of claims 1 to 4 in the preparation of a drug for preventing or treating fatty liver.

11. Use of the pharmaceutical composition according to any one of claims 1 to 4 in the preparation of a drug for preventing or treating cancer.

12. Use of the pharmaceutical composition according to any one of claims 1 to 4 in the preparation of a drug for preventing or treating acne.
